# EUROPEAN PATENT SPECIFICATION

(11) **EP 4 395 465 B1**
(45) Date of publication and mention of the grant of the patent: **04.11.2026**
(21) Application number: 22918091.4
(22) Date of filing: 29.04.2022
(51) Int. Cl.: H05B 47/16, H05B 47/155, H05B 45/20, A61N 5/06

(54) **MODULATION METHOD FOR RHYTHM SPECTRUM**
MODULATIONSVERFAHREN FÜR EIN RHYTHMUSSPEKTRUM
PROCÉDÉ DE MODULATION POUR SPECTRE DE RYTHME

(30) Priority: 06.01.2022 CN 202210013048
(43) Date of publication of application: 03.07.2024
(73) Proprietor: Foshan Electrical and Lighting Co., Ltd, Foshan, Guangdong 528000 (CN)
(72) Inventor: DING, Wenchao, Foshan, Guangdong 528000 (CN); ZHU, Yiguang, Foshan, Guangdong 528000 (CN); WEI, Bin, Foshan, Guangdong 528000 (CN); LU, Zhou, Foshan, Guangdong 528000 (CN)
(74) Representative: Sun, Yiming
(86) International application number: PCT/CN2022/090407
(87) International publication number: WO 2023/130628

(56) References cited:
- WO-A1-2021/030272
- CN-A- 103 493 226
- CN-A- 110 248 443
- CN-A- 112 255 784
- US-A1- 2004 093 045
- US-A1- 2013 250 211
- US-A1- 2016 153 622
- US-A1- 2020 386 613

## Description

### TECHNICAL FIELD

The present disclosure relates to the field of luminescent spectra, and in particular to a method for modulating a rhythmic spectrum.

### BACKGROUND

Indoor lighting environments have a huge influence on health of human bodies. Evidences show that lighting affects visible, physiological and behavioral functions of the human being. The influence of the lighting is associated with intrinsically photosensitive retinal ganglion cells (ipRGCs). Light signals are transmitted from the ipRGCs to the suprachiasmatic nucleus (SCN), thereby adjusting and controlling biological clocks and various physiological activities of the human bodies. If the human bodies receive appropriate light stimulation every day, circadian rhythms in the human bodies can be corrected desirably. Otherwise, the balanced state in the human bodies is disturbed to cause great hazards. The ipRGCs are activated by internal photosensitive proteins called melanopsin. Upon this, a melanopic spectral efficiency function is established to calculate a "melanopic luminous flux", which is then improved as an equivalent melanopic lux (EML). Generally, the EML is used as an evaluation standard to design a rhythmic spectrum.

The rhythm effect of the lighting is mainly affected by a spectral composition, a light intensity, lighting time, etc. It is mostly adjusted by controlling the light intensity and a color temperature. This method is inevitable to affect visual changes, such as the color temperature and a color rendering index (CRI), to reduce a visual effect. As a matter of fact, a great number of evidences show that optimization of the visual efficiency is conventionally reverse to optimization of the rhythmic efficiency. Hence, while the rhythmic efficiency is improved, the visual effect is ensured hardly.

The issue of rhythmic health hasn't been involved universally in existing mainstream indoor lighting lamps. However, according to relevant standards, different requirements are imposed on a rhythmic level of the lighting in different time periods. For example, according to the Shanghai Group Standard T/SIEATA *Ranking Evaluation for Classroom Lighting Quality in Primary and Middle Schools*, the class AAAAA requires that a nighttime EML value is less than or equal to 130, a daytime EML value is greater than or equal to 200, and an eye-level vertical illuminance is greater than or equal to 250 Ix.

Patent US2016/153622A1 describes an LED lamp, whose color temperature is adjustable within 2700-6500K while maintaining a high color rendering index, a low chromaticity difference, and a low manufacturing cost.

Patent US2020/386613A1 relates to illumination devices with an improved emitter module design for calibrating and compensating individual LEDs, so as to accurately maintain a desired luminous flux and a desired chromaticity despite temperature variations, drive current changes, and LED aging.

### SUMMARY

A technical problem to be solved by the present disclosure is to provide a method for modulating a rhythmic spectrum. The present disclosure can provide spectra with a same color temperature and different rhythmic stimulation levels.

To solve the above technical problem, the present disclosure is set out in the appended set of claims 1-5.

The present disclosure has the following beneficial effects:
The present disclosure uses a white LED channel, a blue LED channel, a green LED channel and a blue-green LED channel. By adjusting the luminous flux of each channel, the present disclosure provides two spectra with a same color temperature and different rhythmic stimulation levels, such that an indoor lighting environment provides desirable rhythmic stimulation levels for different time periods without affecting a visual effect.

### BRIEF DESCRIPTION OF THE DRAWINGS

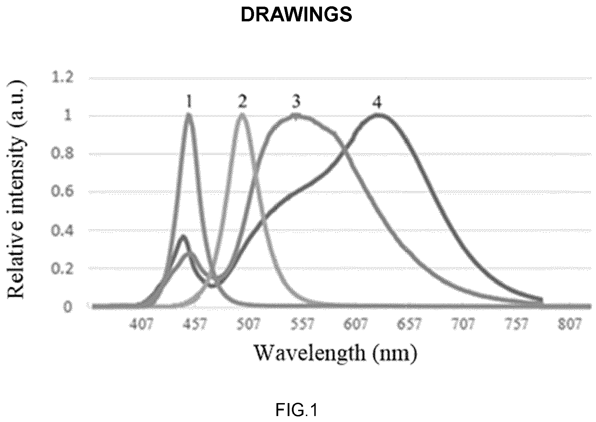

FIG. 1 illustrates spectra of four LEDs according to Embodiments 1-4 of the present disclosure, 1 being a blue LED with a peak wavelength of 455 nm, 2 being a blue-green LED with a peak wavelength of 495 nm, 3 being a green LED with a peak wavelength of 550 nm, and 4 being a white LED with a color temperature of 2,700 K;

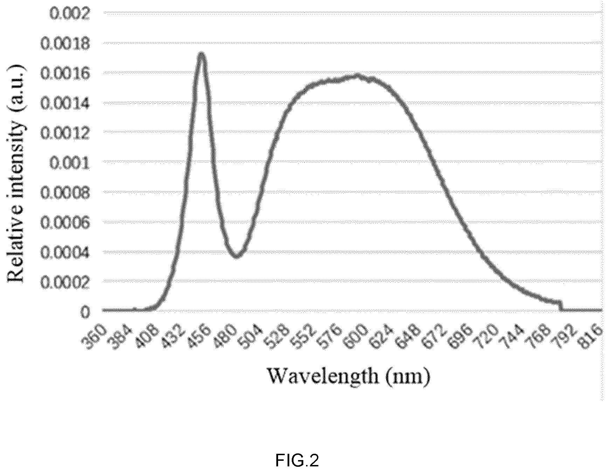

FIG. 2 illustrates a rhythmic spectrum obtained in Embodiment 1 of the present disclosure;

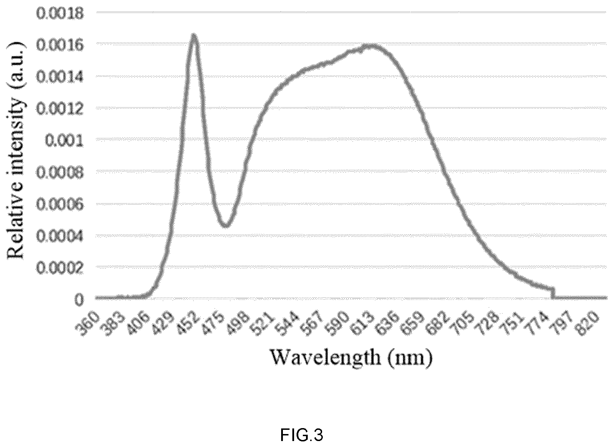

FIG. 3 illustrates a rhythmic spectrum obtained in Embodiment 2 of the present disclosure;

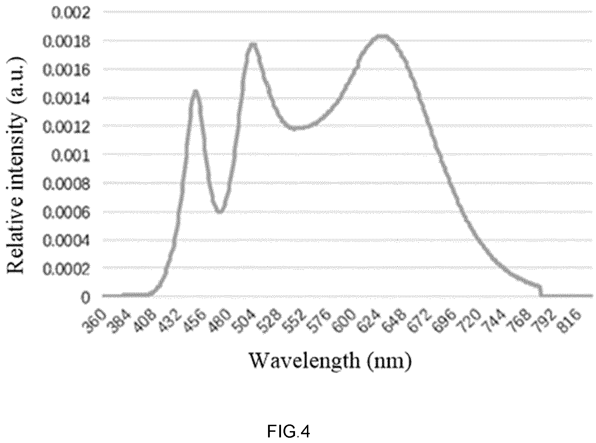

FIG. 4 illustrates a rhythmic spectrum obtained in Embodiment 3 of the present disclosure; and

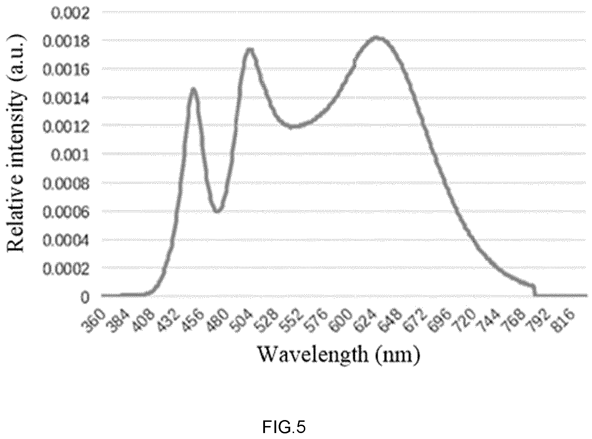

FIG. 5 illustrates a rhythmic spectrum obtained in Embodiment 4 of the present disclosure.

### DETAILED DESCRIPTION

To make the objectives, technical solutions, and advantages of the present disclosure clearer, the present disclosure will be further described in detail below in combination with specific embodiments.

The present disclosure provides a method for modulating a rhythmic spectrum, including the following steps:
(1) A white LED, a blue LED, a green LED and a blue-green LED are provided on a substrate.

The white LED is a common white LED in existing markets. The white LED has a color temperature of 2,600-3,300 K. Exemplarily, the color temperature is 2,650 K, 2,700 K, 2,750 K, 2,800 K, 2,900 K, 3,000 K, 3,100 K or 3,200 K, but is not limited to this. Preferably, the white LED has the color temperature of 2,700 K.

The blue LED has a peak wavelength of 450-465 nm. Exemplarily, the peak wavelength is 450 nm, 454 nm, 456 nm, 458 nm, 460 nm, 462 nm or 464 nm, but is not limited to this. Preferably, the blue LED has the peak wavelength of 455 nm.

The green LED has a peak wavelength of 540-560 nm. Exemplarily, the peak wavelength is 541 nm, 545 nm, 548 nm, 552 nm, 555 nm, 557 nm or 559 nm, but is not limited to this. Preferably, the green LED has the peak wavelength of 550 nm.

The blue-green LED has a peak wavelength of 490-500 nm. Exemplarily, the peak wavelength is 492 nm, 494 nm, 496 nm or 498 nm, but is not limited to this. Preferably, the blue-green LED has the peak wavelength of 495 nm.

The substrate may be a light source plate, but is not limited to this. Preferably, the LEDs are evenly spaced.

S2: A luminous flux of each of the white LED, the blue LED, the green LED and the blue-green LED is adjusted to a preset range, thereby obtaining a rhythmic spectrum with a preset luminous flux.

Specifically, S2 includes:
S21: The luminous flux of each of the white LED, the blue LED, the green LED and the blue-green LED is adjusted.

Specifically, one LED is turned on individually, while other LEDs are turned off. A current is adjusted, an illuminance is measured with an illuminance meter, and the illuminance is used to calculate a corresponding luminous flux. After a required luminous flux is reached, the LED is turned off. The luminous flux of each LED is adjusted according to the above method. It is to be noted that there is no chronological order in adjustment.

Specifically, in an embodiment of the present disclosure, upon completion of the adjustment, a proportion of the luminous flux of each LED to a total luminous flux is as follows:
The luminous flux of the white LED accounts for 43.1-54.1% of the preset luminous flux. Exemplarily, the proportion is 43.5%, 44.2%, 44.3%, 45.3%, 45.9%, 46.4%, 46.8%, 47.5%, 49.3%, 51.5%, 52.4% or 53.7%, but is not limited to this. Preferably, the luminous flux of the white LED accounts for 45.16-54.1% of the total luminous flux.
The luminous flux of the blue LED accounts for 1.1-1.5% of the preset luminous flux. Exemplarily, the proportion is 1.15%, 1.2%, 1.25%, 1.3%, 1.35%, 1.4% or 1.45%, but is not limited to this. Preferably, the luminous flux of the blue LED accounts for 1.3-1.4% of the total luminous flux.
The luminous flux of the green LED accounts for 41.1-55.4% of the preset luminous flux. Exemplarily, the proportion is 42.5%, 44.7%, 47.6%, 51.2%, 51.5%, 51.9%, 52.3%, 53.5%, 53.8%, 54.7% or 55.2%, but is not limited to this. Preferably, the luminous flux of the green LED accounts for 41.1-53.44% of the total luminous flux.
The luminous flux of the blue-green LED accounts for 0-3.5% of the total luminous flux. Exemplarily, the proportion is 0.1%, 0.8%, 1.5%, 1.8%, 2.3%, 2.7%, 2.9% or 3.4%, but is not limited to this.

The LEDs with the above luminous fluxes cooperate to form a rhythmic spectrum with a low rhythmic stimulation level. When the luminous flux is 200 lm, and the color temperature is 4,000 K, EML<130, a ratio of the EML to the illuminance is <0.65, and Ra≥90. The lighting with the weak rhythmic stimulation can make a human body relaxed fully, thereby alleviating a fatigue and maintaining stable internal secretion. This is suitable for scenes such as noon breaks, night activities and evening self-study.

Specifically, in another embodiment of the present disclosure, upon completion of the adjustment, a proportion of the luminous flux of each LED to a total luminous flux is as follows:
The luminous flux of the white LED accounts for 81.08-85.5% of the preset luminous flux. Exemplarily, the proportion is 81.8%, 82.2%, 82.5%, 83.3%, 83.5%, 84.4%, 84.8%, 85.1% or 85.4%, but is not limited to this. Preferably, the luminous flux of the white LED accounts for 81.09-82.56% of the total luminous flux.
The luminous flux of the blue LED accounts for 0.5-1.5% of the preset luminous flux. Exemplarily, the proportion is 0.65%, 0.7%, 0.9%, 1.2%, 1.3%, 1.4% or 1.45%, but is not limited to this. Preferably, the luminous flux of the blue LED accounts for 1.0-1.02% of the total luminous flux.
The luminous flux of the green LED accounts for 1.5-3.8% of the preset luminous flux. Exemplarily, the proportion is 1.52%, 1.65%, 1.69%, 1.75%, 1.83%, 1.98%, 2.32%, 2.55%, 2.89%, 3.02% or 3.54%, but is not limited to this. Preferably, the luminous flux of the green LED accounts for 1.77-3.8% of the total luminous flux.
The luminous flux of the blue-green LED accounts for 12.5-15% of the total luminous flux. Exemplarily, the proportion is 12.7%, 13.3%, 13.5%, 14.2%, 14.6%, 14.8% or 14.9%, but is not limited to this. Preferably, the luminous flux of the blue-green LED accounts for 14.09-14.67% of the total luminous flux.

The LEDs with the above luminous fluxes cooperate to form a rhythmic spectrum with a high rhythmic stimulation level. When the luminous flux is 250 lm, and the color temperature is 4,000 K, EML>200, a ratio of the EML to the illuminance is >0.8, and Ra≥90. The lighting with the strong rhythmic stimulation can make the human body spirited and energetic, thereby greatly improving an efficiency. This is suitable for scenes such as classes, offices and daily homes.

S22: The white LED, the blue LED, the green LED and the blue-green LED are turned on at the same time, thereby obtaining the rhythmic spectrum with the preset luminous flux.

## Claims

1. A method for modulating a rhythmic spectrum, **characterized in that** the method comprises:
(1) providing a white light-emitting diode (LED), a blue LED, a green LED and a blue-green LED on a substrate; and
(2) adjusting a luminous flux of each of the white LED, the blue LED, the green LED and the blue-green LED to a preset range, thereby obtaining a rhythmic spectrum with a preset luminous flux,
wherein the luminous flux of the white LED accounts for 43.1-54.1% of the preset luminous flux, the luminous flux of the blue LED accounts for 1.1-1.5% of the preset luminous flux, the luminous flux of the green LED accounts for 41.1-55.4% of the preset luminous flux, and the luminous flux of the blue-green LED accounts for 0-3.5% of the preset luminous flux; or
the luminous flux of the white LED accounts for 81.09-85.5% of the preset luminous flux, the luminous flux of the blue LED accounts for 0.5-1.5% of the preset luminous flux, the luminous flux of the green LED accounts for 1.5-3.8% of the preset luminous flux, and the luminous flux of the blue-green LED accounts for 12.5-15% of the preset luminous flux;
wherein step (2) comprises:
(2.1) adjusting the luminous flux of each of the white LED, the blue LED, the green LED and the blue-green LED, wherein when the luminous flux of one LED is adjusted, other LEDs are turned off; and
(2.2) turning on the white LED, the blue LED, the green LED and the blue-green LED at the same time, and adjusting to the preset luminous flux, thereby obtaining the rhythmic spectrum with the preset luminous flux;
wherein the white LED has a color temperature of 2,600-3,300 K;
wherein the blue LED has a peak wavelength of 450-465 nm;
wherein the green LED has a peak wavelength of 540-560 nm; and
wherein the blue-green LED has a peak wavelength of 490-500 nm.

2. The method for modulating a rhythmic spectrum according to claim 1, wherein the white LED has the color temperature of 2,700 K, and the blue LED has the peak wavelength of 455 nm.

3. The method for modulating a rhythmic spectrum according to claim 1, wherein the green LED has the peak wavelength of 550 nm, and the blue-green LED has the peak wavelength of 495 nm.

4. The method for modulating a rhythmic spectrum according to claim 1, wherein the luminous flux of the white LED accounts for 45.16-54.1% of the preset luminous flux, the luminous flux of the blue LED accounts for 1.30-1.40% of the preset luminous flux, the luminous flux of the green LED accounts for 41.10-53.44% of the preset luminous flux, and the luminous flux of the blue-green LED accounts for 0-3.5% of the preset luminous flux.

5. The method for modulating a rhythmic spectrum according to claim 1, wherein the luminous flux of the white LED accounts for 81.09-82.56% of the preset luminous flux, the luminous flux of the blue LED accounts for 1.0-1.02% of the preset luminous flux, the luminous flux of the green LED accounts for 1.77-3.80% of the preset luminous flux, and the luminous flux of the blue-green LED accounts for 14.09-14.67% of the preset luminous flux.

## Patentansprüche

1. Verfahren zum Modulieren eines rhythmischen Spektrums, **dadurch gekennzeichnet, dass** das Verfahren Folgendes umfasst:
(1) Bereitstellen einer weißen Leuchtdiode (LED), einer blauen LED, einer grünen LED und einer blaugrünen LED auf einem Substrat; und
(2) Einstellen eines Lichtstroms jeder der weißen LED, der blauen LED, der grünen LED und der blaugrünen LED auf einen voreingestellten Bereich, wodurch ein rhythmisches Spektrum mit einem voreingestellten Lichtstrom erhalten wird,
wobei der Lichtstrom der weißen LED 43,1-54,1 % des voreingestellten Lichtstroms ausmacht, der Lichtstrom der blauen LED 1,1-1,5 % des voreingestellten Lichtstroms ausmacht, der Lichtstrom der grünen LED 41,1-55,4 % des voreingestellten Lichtstroms ausmacht und der Lichtstrom der blaugrünen LED 0-3,5 % des voreingestellten Lichtstroms ausmacht; oder
wobei der Lichtstrom der weißen LED 81,09-85,5 % des voreingestellten Lichtstroms ausmacht, der Lichtstrom der blauen LED 0,5-1,5 % des voreingestellten Lichtstroms ausmacht, der Lichtstrom der grünen LED 1,5-3,8 % des voreingestellten Lichtstroms ausmacht und der Lichtstrom der blaugrünen LED 12,5-15 % des voreingestellten Lichtstroms ausmacht;
wobei Schritt (2) Folgendes umfasst:
(2.1) Einstellen des Lichtstroms jeder der weißen LED, der blauen LED, der grünen LED und der blaugrünen LED, wobei, wenn der Lichtstrom einer LED eingestellt wird, andere LEDs ausgeschaltet sind; und
(2.2) gleichzeitiges Einschalten der weißen LED, der blauen LED, der grünen LED und der blaugrünen LED und Einstellen auf den voreingestellten Lichtstrom, wodurch das rhythmische Spektrum mit dem voreingestellten Lichtstrom erhalten wird;
wobei die weiße LED eine Farbtemperatur von 2.600-3.300 K aufweist;
wobei die blaue LED eine Spitzenwellenlänge von 450-465 nm aufweist;
wobei die grüne LED eine Spitzenwellenlänge von 540-560 nm aufweist; und
wobei die blaugrüne LED eine Spitzenwellenlänge von 490-500 nm aufweist.

2. Verfahren zum Modulieren eines rhythmischen Spektrums nach Anspruch 1, wobei die weiße LED die Farbtemperatur von 2.700 K aufweist und die blaue LED die Spitzenwellenlänge von 455 nm aufweist.

3. Verfahren zum Modulieren eines rhythmischen Spektrums nach Anspruch 1, wobei die grüne LED die Spitzenwellenlänge von 550 nm aufweist und die blaugrüne LED die Spitzenwellenlänge von 495 nm aufweist.

4. Verfahren zum Modulieren eines rhythmischen Spektrums nach Anspruch 1, wobei der Lichtstrom der weißen LED 45,16-54,1 % des voreingestellten Lichtstroms ausmacht, der Lichtstrom der blauen LED 1,30-1,40 % des voreingestellten Lichtstroms ausmacht, der Lichtstrom der grünen LED 41,10-53,44 % des voreingestellten Lichtstroms ausmacht und der Lichtstrom der blaugrünen LED 0-3,5 % des voreingestellten Lichtstroms ausmacht.

5. Verfahren zum Modulieren eines rhythmischen Spektrums nach Anspruch 1, wobei der Lichtstrom der weißen LED 81,09-82,56 % des voreingestellten Lichtstroms ausmacht, der Lichtstrom der blauen LED 1,0-1,02 % des voreingestellten Lichtstroms ausmacht, der Lichtstrom der grünen LED 1,77-3,80 % des voreingestellten Lichtstroms ausmacht und der Lichtstrom der blaugrünen LED 14,09-14,67 % des voreingestellten Lichtstroms ausmacht.

## Revendications

1. Procédé de modulation d'un spectre rythmique, **caractérisé en ce que** le procédé comprend :
(1) la fourniture d'une diode électroluminescente (LED) blanche, d'une LED bleue, d'une LED verte et d'une LED bleu-vert sur un substrat ; et
(2) le réglage d'un flux lumineux de chacune de la LED blanche, de la LED bleue, de la LED verte et de la LED bleu-vert dans une plage prédéfinie, ce qui permet d'obtenir un spectre rythmique présentant un flux lumineux prédéfini,
dans lequel le flux lumineux de la LED blanche représente 43,1-54,1 % du flux lumineux prédéfini, le flux lumineux de la LED bleue représente 1,1-1,5 % du flux lumineux prédéfini, le flux lumineux de la LED verte représente 41,1-55,4 % du flux lumineux prédéfini, et le flux lumineux de la LED bleu-vert représente 0-3,5 % du flux lumineux prédéfini ; ou
le flux lumineux de la LED blanche représente 81,09-85,5 % du flux lumineux prédéfini, le flux lumineux de la LED bleue représente 0,5-1,5 % du flux lumineux prédéfini, le flux lumineux de la LED verte représente 1,5-3,8 % du flux lumineux prédéfini, et le flux lumineux de la LED bleu-vert représente 12,5-15 % du flux lumineux prédéfini ;
dans lequel l'étape (2) comprend :
(2.1) le réglage du flux lumineux de chacune de la LED blanche, de la LED bleue, de la LED verte et de la LED bleu-vert, dans lequel, lorsque le flux lumineux d'une LED est réglé, les autres LED sont éteintes ; et
(2.2) l'allumage simultané de la LED blanche, de la LED bleue, de la LED verte et de la LED bleu-vert, et le réglage au flux lumineux prédéfini, ce qui permet d'obtenir le spectre rythmique présentant le flux lumineux prédéfini ;
dans lequel la LED blanche a une température de couleur de 2.600-3.300 K ;
dans lequel la LED bleue a une longueur d'onde de pic de 450-465 nm ;
dans lequel la LED verte a une longueur d'onde de pic de 540-560 nm ; et
dans lequel la LED bleu-vert a une longueur d'onde de pic de 490-500 nm.

2. Procédé de modulation d'un spectre rythmique selon la revendication 1, dans lequel la LED blanche a la température de couleur de 2.700 K, et la LED bleue a la longueur d'onde de pic de 455 nm.

3. Procédé de modulation d'un spectre rythmique selon la revendication 1, dans lequel la LED verte a la longueur d'onde de pic de 550 nm, et la LED bleu-vert a la longueur d'onde de pic de 495 nm.

4. Procédé de modulation d'un spectre rythmique selon la revendication 1, dans lequel le flux lumineux de la LED blanche représente 45,16-54,1 % du flux lumineux prédéfini, le flux lumineux de la LED bleue représente 1,30-1,40 % du flux lumineux prédéfini, le flux lumineux de la LED verte représente 41,10-53,44 % du flux lumineux prédéfini, et le flux lumineux de la LED bleu-vert représente 0-3,5 % du flux lumineux prédéfini.

5. Procédé de modulation d'un spectre rythmique selon la revendication 1, dans lequel le flux lumineux de la LED blanche représente 81,09-82,56 % du flux lumineux prédéfini, le flux lumineux de la LED bleue représente 1,0-1,02 % du flux lumineux prédéfini, le flux lumineux de la LED verte représente 1,77-3,80 % du flux lumineux prédéfini, et le flux lumineux de la LED bleu-vert représente 14,09-14,67 % du flux lumineux prédéfini.
